(19) **Europäisches Patentamt**

**European Patent Office**

**Office européen des brevets**

(11) **EP 1 151 742 A2**

(12) **DEMANDE DE BREVET EUROPEEN**

(43) Date de publication:
**07.11.2001 Bulletin 2001/45**

(51) Int Cl.$^{7}$: **A61K 7/02**

(21) Numéro de dépôt: **01400866.8**

(22) Date de dépôt: **04.04.2001**

| (84) Etats contractants désignés:<br>**AT BE CH CY DE DK ES FI FR GB GR IE IT LI LU MC NL PT SE TR**<br>Etats d'extension désignés:<br>**AL LT LV MK RO SI** | (72) Inventeur: **Afriat, Isabelle**<br>**75003 Paris (FR)** |
| :--- | :--- |
| (30) Priorité: **04.05.2000 FR 0005712** | (74) Mandataire: **Rasson, Catherine et al**<br>**L'OREAL-DPI**<br>**6 rue Bertrand Sincholle**<br>**92585 Clichy Cedex (FR)** |
| (71) Demandeur: **L'OREAL**<br>**75008 Paris (FR)** | |

(54) **Utilisation de fibres dans une composition de soin ou de maquillage pour matifier la peau**

(57)     La présente demande concerne l'utilisation de fibres dans une composition de soin ou de maquillage de la peau, pour matifier, lisser et/ou uniformiser le teint, et/ou pour estomper les défauts du relief de la peau.

Les fibres sont en particulier des fibres de polyamide ayant une longueur de 1 μm à 10 mm et un facteur de forme de 5 à 150.

La composition utilisée donne sur la peau un indice de couvrance supérieur à 0,1 et de préférence supérieur à 0,13.

L'invention se rapporte aussi à un procédé de traitement cosmétique en vue de matifier, lisser et/ou uniformiser le teint de la peau, et/ou d'estomper les microreliefs, les rides, les ridules, les pores de la peau, comprenant l'application sur la peau, de fibres dans une composition cosmétique de soin de la peau.

EP 1 151 742 A2

**Description**

La présente demande concerne l'utilisation de fibres dans une composition de soin ou de maquillage de la peau, pour matifier, lisser et/ou uniformiser le teint. Elle se rapporte aussi à un procédé de traitement cosmétique en vue de matifier, lisser et/ou uniformiser le teint de la peau, et/ou d'estomper les microreliefs, les rides, les pores de la peau, comprenant l'application sur la peau, d'une composition cosmétique contenant des fibres.

Les compositions de soin de la peau ou de maquillage ayant des propriétés matifiantes sont généralement utilisées pour résoudre les problèmes de brillance occasionnés par un excès de sébum et pour améliorer la tenue du maquillage à long terme, le maquillage ayant tendance à se dégrader visuellement au cours de la journée. Elles donnent un aspect mat à la peau, résultant d'un pouvoir diffusant de la lumière à la surface de la peau. Elles peuvent aussi être utilisées pour estomper les défauts de la peau tels que les microreliefs, les rides, les ridules, les pores ou les variations de couleur.

Les compositions classiques dites matifiantes contiennent généralement très peu de corps gras ou sont dépourvues de corps gras. Elles sont généralement constituées de poudres absorbant le sébum et l'huile excédentaire de la composition non absorbée par la peau. Parmi les poudres matifiantes d'origine naturelle ou synthétique, on peut citer notamment les charges telles que le talc, l'amidon, le mica, la silice, les poudres de nylon, les poudres de polyéthylène, la poly-beta-alanine, les poly(méthacrylate de méthyle). Ce type de charges présente l'inconvénient de ne pas apporter à la peau un aspect naturel en donnant un aspect poudreux voire plâtreux, et d'accentuer les défauts de la peau. De plus, les compositions les contenant sont généralement desséchantes à long terme et s'étalent difficilement, Leur effet matifiant est peu durable dans le temps.

Le document EP-A-502769 décrit des compositions matifiantes apportant une couche translucide et un aspect naturel à la peau maquillée. Il s'agit de dispersions de particules sphériques dans un liant gras dans un rapport en poids charges/liant très spécifique. Pour avoir un effet matifiant, il faut une forte proportion de poudres et, de ce fait, ces compositions peuvent être desséchantes. En outre, elles ont tendance à pelucher lors de l'étalement et à donner un effet blanchissant à la peau en raison de la forte concentration en poudres.

La Demanderesse a découvert de manière surprenante que les fibres, et notamment les fibres de polyamide, pouvaient constituer un agent matifiant remarquable. Les compositions cosmétiques contenant ces fibres permettent de matifier, lisser et/ou uniformiser le teint, tout en étant douces à l'application, faciles à étaler, non collantes et non desséchantes pour la peau. Elles présentent une bonne efficacité de couvrance sur la peau et peuvent être utilisées en particulier pour estomper les défauts du relief de la peau tels que les microreliefs, les rides, les pores, tout en conférant à celle-ci un aspect naturel.

Il est connu par exemple par le document JP07-196440 des compositions cosmétiques contenant des fibres de polyamide courtes, celles-ci donnant aux dites compositions un toucher velouté et une bonne tenue cosmétique. Toutefois, aucun document ne décrit que les fibres puissent avoir un effet matifiant et/ou couvrant ni qu'elles permettent d'estomper les défauts (rides, ridules, ou pores) de la peau (effet de couvrance).

Aussi, la présente invention a pour objet l'utilisation cosmétique de fibres dans une composition de soin de la peau, comme agent destiné à matifier, lisser et/ou uniformiser le teint, et/ou à estomper les défauts du relief de la peau.

L'invention a encore pour objet l'utilisation cosmétique de fibres dans une composition de soin de la peau, destinée à matifier, lisser et/ou uniformiser le teint, et/ou à estomper les défauts du relief de la peau.

L'invention a aussi pour objet l'utilisation cosmétique de fibres dans une émulsion cosmétique, comme agent destiné à matifier, lisser et/ou uniformiser le teint, et/ou à estomper les défauts du relief de la peau, et l'utilisation cosmétique de fibres dans une émulsion cosmétique destinée à matifier, lisser et/ou uniformiser le teint, et/ou à estomper les défauts du relief de la peau.

L'invention a encore pour objet l'utilisation cosmétique de fibres dans une composition de soin et/ou de maquillage de la peau, pour estomper les microreliefs, les rides, les ridules, les pores ou les variations de couleur.de la peau.

L'invention a aussi pour objet un procédé de traitement cosmétique en vue de matifier, lisser et/ou uniformiser le teint, et/ou d'estomper les microreliefs, les rides, les ridules, les pores de la peau, comprenant l'application sur la peau, d'une composition cosmétique de soin de la peau, contenant des fibres dans un milieu physiologiquement acceptable.

L'invention se rapporte en outre à un procédé de maquillage des microreliefs, rides, ridules et/ou pores de la peau, consistant à appliquer sur la peau, une composition cosmétique contenant des fibres dans un milieu physiologiquement acceptable.

La composition utilisée selon l'invention contient les fibres en une quantité suffisante pour produire un indice de couvrance supérieur à 0,1 et de préférence supérieur à 0,13. L'indice de couvrance est défini comme étant le rapport Yn/Yb (Clarté du noir/clarté du blanc) dans le test de couvrance décrit ci-dessous.

L'invention a encore pour objet l'utilisation cosmétique de fibres dans une composition de soin ou de maquillage de la peau, en une quantité suffisante pour que la dite composition ait un indice de couvrance supérieur à 0,1.

La composition cosmétique contenant les fibres contient un milieu physiologiquement acceptable. On entend ici par « milieu physiologiquement acceptable », un milieu compatible avec la peau, les lèvres, le cuir chevelu, cils, les yeux, les ongles et/ou les cheveux.

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 μm (0,001 mm) à 10 mm, de préférence de 0,1 μm à 5 mm et mieux de 0,1 mm à 1,5 mm. Leur section peut être comprise dans un cercle de diamètre (D) allant de 1 nm (0,001 μm) à 100 μm, de préférence allant de 1 nm (0,001 μm) à 50 μm et mieux de 5 μm à 40 μm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de laine, de lin, de cellulose extraites notamment du bois, des légumes ou des algues, de polyamide (Nylon®), de cellulose modifiée (rayonne, viscose, acétate notamment d'acétate de rayonne), de poly-p-phénylène téréphtalamide notamment de Kevlar® , en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/ polyester.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

On peut utiliser un mélange de plusieurs sortes de fibres.

Selon leurs propriétés, les fibres utilisées selon la présente invention peuvent être introduites dans un milieu aqueux, un milieu huileux ou dans une poudre.

Les fibres utilisables dans la composition selon l'invention sont de préférence choisies parmi les fibres de polyamide, les fibres de poly-p-phénylène téréphtalamide, les fibres de coton et leurs mélanges. Leur longueur peut aller de 0,1 à 10 mm, de préférence de 0,1 à 1 mm, leur diamètre moyen peut aller de 5 à 50 μm et le facteur de forme va de préférence de 5 à 150.

En particulier, on peut utiliser les fibres de polyamide commercialisées par les Etablissements P. Bonte sous le nom Polyamide 0,9 Dtex 0,3 mm, ayant un diamètre moyen de 15 à 20 μm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 μm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut encore utiliser des fibres de coton ayant un diamètre moyen de 20 μm, une longueur de 0,3 mm, et un facteur de forme de 15, telles que celles commercialisées par la société Filature de Lomme, par la société Textiles des dunes ou par la société Velifil.

Les fibres peuvent être présentes dans la composition selon l'invention en une quantité allant de 0,1 à 30 % en poids, de préférence de 1 à 25 % en poids et mieux de 5 à 25 % en poids par rapport au poids total de la composition. La quantité de fibres à utiliser dépend de la nature et de la forme de section des fibres utilisées. Ainsi, pour les fibres de polyamide, une quantité d'au moins 5 % de fibres permet d'obtenir un effet matifiant et un estompage des défauts de la peau, particulièrement satisfaisants. Pour les microfibrilles, une quantité de 1 % donne déjà de bons résultats.

L'indice de couvrance permet de caractériser l'effet de couvrance d'une composition. Pour déterminer l'indice de couvrance, on prépare une carte de contraste en étalant la composition en une épaisseur de 150 μm, sur une plaque de contraste PENOPAC 1A - BYK-Gardner (140 x 254 mm.) grâce à un applicateur multiple 4 faces 50/100/150/300 microns. On laisse sécher le film ainsi obtenu pendant environ 24 heures.

On étalonne un colorimètre CR 200 Minolta grâce à une plaque blanche de calibration spéciale (Y= 92 ; x= 0,3138 ; y= 0,3193). Le diagramme CIE (Comité International de l'Eclairage) des coordonnées trichromatiques permet de caractériser toutes les couleurs au moyen de coordonnées planes, x et y qui définissent la chromaticité, c'est-à-dire l'ensemble teinte et saturation, et d'une troisième coordonnée Y définissant la clarté. L'appareil est programmé pour réaliser 3 prises à chaque mesure et calcule automatiquement la moyenne. On réalise 2 mesures sur chaque partie de la carte de contraste (noire/blanche), et on mesure les valeurs de Yn (Y noire) et de Yb (Y blanche).

L'indice de couvrance correspond au rapport Yn/Yb. Plus ce rapport est proche de 1, plus l'effet est couvrant. Pour un indice de couvrance de 1, la couvrance est totale, c'est-à-dire que l'on obtient un effet blanc sur la peau, ce qui n'est pas souhaitable pour avoir un aspect naturel de l'effet matifiant. Pour avoir une couvrance satisfaisante et un bon effet matifiant selon la présente invention, l'indice de couvrance doit être supérieur à 0,1, et de préférence aller de 0,1 à 0,5 et mieux de 0,13 à 0,4.

Les compositions selon l'invention contenant les fibres peuvent se présenter sous toutes les formes galéniques classiquement utilisées pour une application topique et notamment sous forme de compositions anhydres, de gels huileux, de solutions aqueuses ou hydroalcooliques, de dispersions du type lotion ou sérum, de gels aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de microcapsules, de microparticules ou de dispersions vésiculaires de type ionique et/ou non ionique. Ces compositions sont préparées selon les méthodes usuelles.

Selon un mode particulier de réalisation de l'invention, la composition contenant les fibres est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (polyisobutène hydrogénée), les huiles de silicone non volatiles ou volatiles (cyclométhicones telles que cyclopentasiloxane) et les huiles fluorées (perfluoropolyéthers). On peut aussi utiliser, comme matières grasses, des alcools gras, des acides gras, des cires telles que la cire microcristalline, la cire de jojoba, la cire de lanoline et la cire d'abeille. La phase huileuse de l'émulsion peut contenir aussi des gommes telles que les gommes de silicone (diméthiconol), des résines et notamment les résines de silicone telles que la trifluorométhyl C1-4 alkyldimethicone, et les élastomères de silicone tels que les produits commercialisés sous la dénomination "KSG" par la société Shin-Etsu, sous la dénomination "Trefil" par la société Dow Corning ou sous la dénomination "Gransil" par la société Grant Industries.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90[R] par la société Goldschmidt.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques. Ces adjuvants ainsi que leurs concentrations doivent être tels qu'ils ne modifient pas la propriété recherchée pour la composition de l'invention.

Comme solvants utilisables dans l'invention, on peut citer les alcools inférieurs comportant de 1 à 4 atomes de carbone, notamment l'éthanol et l'isopropanol, le propylène glycol.

Comme gélifiants lipophiles, on peut citer les argiles modifiées telles que le l'hectorite et ses dérivés, comme les produits commercialisés sous les noms de Bentone.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 40% en poids et de préférence de 1 à 10 % en poids par rapport au poids total de la composition.

Du fait de l'effet mat obtenu avec la composition utilisée selon l'invention, cette composition peut notamment être utilisée pour le traitement des peaux grasses, en particulier pour réguler l'aspect huileux de la peau. Elle peut alors contenir de manière avantageuse au moins un actif de traitement des peaux grasses. Cet actif peut notamment être choisi parmi les dérivés de b-lactame, les dérivés de quinolone, la ciprofloxacine, la norfloxacine, la tétracycline et ses sels, l'érythromycine et ses sels, l'amikacine et ses sels, le 2,4,4'-trichloro-2'-hydroxy diphényl éther (ou triclosan), le 3,4,4'-trichlorocarbanilide (ou triclocarban), le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamidine et ses sels, la gentamicine et ses sels, la kanamycine et ses sels, la linéomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amanfadine, le para-chloro-méta-xylénol, la nystatine, le tolnaftate, l'acide 3-hydroxy benzoïque et ses sels, l'acide 4-hydroxy benzoïque et ses sels, l'acide 2-hydroxybutanoïque et ses sels, l'acide 2-hydroxypentanoïque et ses sels, l'acide 2-hydroxyhexanoïque et ses sels, l'acide phytique et ses sels, l'acide N-acétyl-L-cystéine et ses sels, l'acide lipoïque et ses sels, l'acide azélaïque et ses sels, l'acide arachidonique et ses sels, l'ibuprofène, le naproxène, l'hydrocortisone, l'acétominophène, le résorcinol, l'octopirox, le chlorhydrate de lidocaïne, le clotrimazole, l'acide 10-hydroxy-2-décanoïque et ses sels, les sels de zinc tels que le gluconate de zinc, les extraits végétaux de la famille des éricacées tels que les extraits de gaulthérie ou *Gaultheria procumbens),* les extraits végétaux riches en soufre tels que les extraits de Lamier blanc ou *Lamium album*), et leurs mélanges.

Ce ou ces actifs peuvent être présents en une quantité allant par exemple de 0,0001 à 30 % en poids, de préférence de 0,001 à 20 % en poids et mieux de 0,01 à 15 % en poids par rapport au poids total de la composition.

Aussi, la présente invention a aussi pour objet une composition cosmétique comprenant des fibres et au moins un actif de traitement des peaux grasses, notamment choisi parmi ceux indiqués ci-dessus, et à l'utilisation cosmétique d'une telle composition pour réguler l'aspect huileux de la peau et/ou traiter les peaux grasses.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook), et les quantités en pourcentage en poids sauf mention contraire.

**Exemple 1 : Emulsion E/H**

| | |
|---|---|
| (1) Cyclomethicone/Disteardimonuim Hectorite/alcool (Mélange 85/10/5 vendu sous le nom Bentone Gel VS-5V par Elementis Specialties) | 3 % |
| (2) Chlorure de sodium | 0,7 % |
| (3) Trifluoromethyl C1-4 alkyldimethicone (FL-5 (X-22-819) vendu par Shin Etsu) | 4 % |
| (4) Nylon 12 (Orgasol 2002 Extra D Nat Cos vendu par Atochem) | 1,5 % |
| (5) Fibres de polyamide (Polyamide 0.9dtex, 0,3mm - société Paul Bonte) | 12 % |
| (6) Cyclomethicone / dimethicone copolyol (DC-5225 C vendu par Dow Corning) | 10 % |
| (7) Dimethicone / Dimethiconol (DC 1503 vendu par Dow Corning) | 2,5 % |
| (8) Cyclopentasiloxane | 7 % |

(suite)

| | |
|---|---|
| (9) Conservateurs | 1 % |
| (10) Glycérine | 5 % |
| (11) Ethanol | 5 % |
| (12) Eau | qsp 100 % |

Mode opératoire : on mélange à la spatule les constituants (3), (7), (1), la moitié du cyclopentasiloxane (8) et les fibres (5), puis on passe le mélange à la tri-cylindre 2 fois. On mélange par ailleurs le DC-5225C avec l'autre moitié de cyclopentasiloxane, on mélange avec le mélange précédemment obtenu, et on homogénéise pour obtenir la phase huileuse.

Par ailleurs, on prépare la phase aqueuse par mélange des constituants de cette phase : eau, glycérine, éthanol, sel, conservateurs. On fait l'émulsion en versant peu à peu la phase aqueuse dans la phase huileuse sous agitation.

La composition ainsi obtenue possède des propriétés matifiantes qui persistent dans le temps, et elle donne un aspect naturel à la peau après application.

Test pour déterminer l'indice de couvrance :

On a déterminé l'indice de couvrance de la composition de l'exemple 1 et de compositions analogues ne contenant pas de fibres (placebo) ou contenant une quantité moindre de fibres :

1) Compositions

| Compositions | Placébo | Ex. avec 6% de fibres | Ex. avec 8% de fibres | Ex. avec 10% de fibres | Ex. avec 12% de fibres |
|---|---|---|---|---|---|
| Trifluoromethyl C1-4 alkyldimethicone | 4,5 | 4,3 | 4,2 | 4,1 | 4,0 |
| DC 1503 | 2,8 | 2,7 | 2,6 | 2,6 | 2,5 |
| Bentone Gel VS-5V | 3,4 | 3,2 | 3,1 | 3,1 | 3,0 |
| Nylon 12 | 1,7 | 1,6 | 1,6 | 1,5 | 1,5 |
| Fibres de polyamide | | 6,0 | 8,0 | 10,0 | 12,0 |
| | | | | | |
| Cyclopentasiloxane | 8,0 | 7,5 | 7,3 | 7,2 | 7,0 |
| DC-5225 C | 11,4 | 10,7 | 10,5 | 10,2 | 10,0 |
| | | | | | |
| Chlorure de sodium | 0,8 | 0,7 | 0,7 | 0,7 | 0,7 |
| Glycérine | 5,7 | 5,3 | 5,2 | 5,1 | 5,0 |
| Ethanol | 5,7 | 5,3 | 5,2 | 5,1 | 5,0 |
| Conservateurs | 1,1 | 1,1 | 1,0 | 1,0 | 1,0 |
| Eau | qsp 100,0 | qsp 100,0 | qsp 100,0 | qsp 100,0 | qsp 100,0 |

2)-Résultats

| Valeurs obtenues | Placébo | Ex. avec 6% de fibres | Ex. avec 8% de fibres | Ex. avec 10% de fibres | Ex. avec 12% de fibres |
|---|---|---|---|---|---|
| moyenne $Y_{noire}$ | 4,2600 | 10,9500 | 17,2800 | 24,0000 | 27,1600 |
| moyenne $X_{noire}$ | 0,3100 | 0,2940 | 0,2962 | 0,2987 | 0,3001 |
| moyenne $y_{noire}$ | 0,3170 | 0,3019 | 0,3040 | 0,3062 | 0,3073 |
| moyenne $Y_{blanche}$ | 82,4700 | 82,4800 | 82,2400 | 82,4100 | 82,4700 |
| moyenne $X_{blanche}$ | 0,3166 | 0,3168 | 0,3169 | 0,3168 | 0,3167 |
| moyenne $y_{blanche}$ | 0,3222 | 0,3224 | 0,3225 | 0,3223 | 0,3222 |
| $Y_{noire}/Y_{blanche}$ | 0,0517 | 0,1328 | 0,2101 | 0,2912 | 0,3293 |

Il ressort du tableau ci-dessus que l'indice de couvrance n'est supérieur à 0,1 que pour les compositions contenant des fibres et qu'il augmente avec le pourcentage de fibres contenues dans la formule.

**Exemple 2 : Emulsion E/H**

| | |
|---|---|
| (1) Cire microcristalline | 1,41 % |
| (2) Polyisobutène hydrogéné | 5,44 % |
| (3) Propylparaben (conservateur) | 0,02 % |
| (4) Polyaminopropyl biguanide (conservateur) | 1 % |
| (5) Sulfate de magnésium | 0,7 % |
| (6) Silice | 0,64 % |
| (7) Fibres de polyamide (Polyamide 0,9 Dtex, 0,3 mm - Société Paul Bonte) | 8,2 % |
| (8) Copolymère éthylène/acide acrylique | 0,7 % |
| (9) Acrylates copolymer | 0,05 % |
| (10) Cyclopentasiloxane | 20 % |
| (11) Cyclomethicone/dimethicone copolyol (DC-5225C vendu par Dow Corning) | 10 % |
| (12) Polymethylsesquioxane | 0,5 % |
| (13) Dimethicone/vinyl dimethicone crosspolymer/dimethicone (KSG 16 vendu par Shin-Etsu) | 3 % |
| (14) Glycérine | 5 % |
| (15) Eau | qsp 100 % |

Mode opératoire : on mélange les constituants (1), (2), (3), (6), (8), (9), (12) et (13). On y ajoute les fibres (7) et on mélange bien. On ajoute ensuite les constituants (10) et (11) et on homogénéise la phase huileuse ainsi obtenue. On fait l'émulsion au moritz en versant la phase aqueuse obtenue par mélange des constituants (4), (5), (14) et (15), dans la phase huileuse précédemment obtenue.

La composition obtenue est apte à matifier la peau et à en camoufler les imperfections (rides et ridules)

Test d'efficacité de matité: Pour mettre en évidence l'effet matifiant de la composition selon l'invention, on a évalué la brillance de la peau au cours du temps après 15 minutes et 1 heure, observée sur un échantillon de 17 personnes à peau grasse et brillante, sur lesquelles on a appliqué la composition matifiante de l'exemple 2 et, pour comparaison, une composition identique ne contenant pas de fibres.

On applique la composition matifiante sur chaque personne, à raison de 2 mg/cm$^2$ en une application unique sur un demi-front, l'autre moitié servant de zone témoin avec une application unique sur un demi-front de la composition ne contenant pas de fibres. Une randomisation est effectuée pour éviter les effets de zone.

Les conditions climatiques sont les suivantes :

Température : 22°C

Humidité relative : 41%

On mesure aux temps T= 0 ($T_0$), T= 15 mn, T=1h, la brillance de la surface de la peau maquillée (ou non traitée pour la zone témoin) au moyen d'un appareil de mesure décrit dans la demande publiée FR-2,650,890 et à partir des paramètres de réflexion parallèle et de réflexion croisée propres à ce dispositif et permettant d'évaluer la brillance de la surface de la peau.

On calcule dans un premier temps, au temps T, la variation de la brillance moyenne mesurée sur la zone traitée par la formule :

$$\Delta_1 = B_T - B_o/B_o$$

où :

$B_0$ désigne la brillance moyenne mesurée à $T_0$
$B_T$ désigne la brillance moyenne mesurée à T

On calcule dans un second temps, au temps T, la variation de la brillance moyenne mesurée sur la zone témoin (traitée par la composition ne contenant pas de fibre) par la formule :

$$\Delta_2 = B'_T - B'_o/B'_o$$

où :

$B'_0$ désigne la brillance moyenne mesurée sur la zone témoin à $T_0$
$B'_T$ désigne la brillance moyenne mesurée sur la zone témoin à T

Les résultats de ces tests sont résumés dans le tableau suivant :

| Temps | 15 minutes | 1 heure |
|---|---|---|
| Variation de la brillance moyenne sur zone traitée (exemple 2) $\Delta_1$ | - 14% | - 13% |
| Variation de la brillance moyenne sur zone témoin (composition sans fibres) $\Delta_2$ | - 4% | - 4% |

Ces résultats montrent que les fibres donnent aux compositions les contenant de bonnes propriétés matifiantes sur la peau.

**Exemple 3 : Produit coulé**

*Phase A1*

- Cire de jojoba    5,3 %
- Polyglyceryl-4 isostéarate/Cetyl dimethicone copolyol / hexyl laurate (Abil WE 09 vendu par Goldschmidt)    2,2 %
- Cetearyl octanoate / isoproyl myristate    3,1 %
- Polyethylene    0,7 %

*Phase A2*

- Cyclohexasiloxane    3,1 %

*Phase B*

- Chlorure de sodium        0,5 %
- Glycérine        2 %
- Conservateurs        1 %
- Eau        qsp 100 %

*Phase C*

- Cyclomethicone/Disteardimonuim Hectorite/ alcool (Mélange 85/10/5 vendu sous le nom Bentone Gel VS-5V par Elementis Specialties)        1,1 %
- Trifluoromethyl C1-4 alkyldimethicone (FL-5 (X-22-819) vendu par Shin Etsu)        2,1 %
- Nylon 12 (Orgasol 2002 Extra D Nat Cos vendu par Atochem)        1 %
- Fibres de polyamide (Polyamide 0.9dtex, 0,3mm - Société Paul Bonte)        8 %
- Dimethicone / Dimethiconol (DC 1503 vendu par Dow Corning)        1,3 %

Mode opératoire : on prépare la phase C en mélangeant tous les constituants et en passant le mélange tois fois à la tri-cylindre. On met la phase aqueuse (B) (chlorure de sodium + glycerine + eau + conservateurs) au bain-marie à 90°C. On fait fondre la phase A1 en remuant le mélange des constituants à la spatule à 80-85°C (jusqu'à liquéfaction complète). On ajoute ensuite la phase A2 à la phase A1, puis on place le mélange au bain-marie à 80-85°C en maintenant l'agitation.

On fait l'émulsion en versant la phase B dans le mélange de A1 et de A2, à 85°C sous agitation. On y ajoute ensuite la phase C vers 70-75°C en mélangeant environ 2 minutes toujours sous agitation.

Dès que l'émulsion est faite, on verse dans des coupelles à chaud et on laisse refroidir.

On obtient une composition de soin sous forme d'un produit coulé compact qui s'applique avec une éponge.

La composition a été testée sur un panel de 5 personnes. Elle a été trouvée de texture agréable, facile à étaler, confortable, laissant, après application, la peau douce, non collante. En outre, il a été noté un effet éclaircissant, lissant et matifiant de la peau traitée.

**Exemple 4 : Poudre**

- Talc        75,3 %
- Fibres de polyamide - Polyamide 0.9dtex, 0,3mm (Sté Paul Bonte)        7,5 %
- Poudre de nylon        10 %
- Pigments        3 %
- Liant silicone        4 %
- Conservateur        0,2 %

Mode opératoire: L'ensemble des composés, à l'exception des fibres, sont mélangés dans un appareil Baker Perkins. Le liant silicone est un mélange de polyméthyl/cétyldiméthylsiloxane (Abil Wax 9801 de la société Goldschmidt), de polydiméthylsiloxane/résine triméthylsiloxysilicate (Dow Corning Fluid 593) et de polydiméthylsiloxane 10 cSt (Dow Corning Fluid 200). Les fibres sont ajoutées sous agitation en fin de préparation dans un broyeur type Hosokawa Alpine.

La composition obtenue présente au maquillage sur un panel de 16 femmes :

- un reflet mat/satiné,
- une bonne uniformité,
- un effet correcteur

**Exemple 5 : Rouge à lèvres**

- Cire de lanoline          5 %
- Cire microcristalline          11 %
- Cire d'abeille modifiée          4,5 %
- Huile d'arara          21 %
- Huile de sésame          22 %
- Argile modifiée          0,6 %
- Lanoline acétylée          6 %
- Fibre de coton (0,3 mm de long)          5 %
- Pigments          9 %
- Anti-oxydant          0,1 %
- Lanoline qsp          100 %

On obtient un stick de rouge à lèvres, homogène et brillant. A l'application sur un panel de 24 personnes, ce film de rouge à lèvres conduit à une diminution significative de la brillance comparativement au film obtenu avec un rouge à lèvres ne contenant pas de fibres.


**Revendications**

1. Utilisation cosmétique de fibres dans une composition de soin de la peau, comme agent destiné à matifier, lisser et/ou uniformiser le teint, et/ou à estomper les défauts du relief de la peau.

2. Utilisation cosmétique de fibres dans une composition de soin de la peau, destinée à matifier, lisser et/ou uniformiser le teint, et/ou à estomper les défauts du relief de la peau.

3. Utilisation cosmétique de fibres dans une émulsion cosmétique, comme agent destiné à matifier, lisser et/ou uniformiser le teint, et/ou à estomper les défauts du relief de la peau.

4. Utilisation cosmétique de fibres dans une émulsion cosmétique destinée à matifier, lisser et/ou uniformiser le teint, et/ou à estomper les défauts du relief de la peau.

5. Utilisation cosmétique de fibres dans une composition de soin et/ou de maquillage de la peau, pour estomper les microreliefs, les rides, les ridules, les pores ou les variations de couleur.de la peau.

6. Utilisation cosmétique de fibres dans une composition de soin ou de maquillage de la peau, en une quantité suffisante pour que la dite composition ait un indice de couvrance supérieur à 0,1.

7. Utilisation selon l'une quelconque des revendications 1 à 6, **caractérisée en ce que** les fibres ont une longueur (L) allant de 1 µm à 10 mm.

8. Utilisation selon l'une quelconque des revendications précédentes, **caractérisée en ce que** les fibres ont une section comprise dans un cercle de diamètre (D) allant de 1 nm à 100 µm.

9. Utilisation selon l'une quelconque des revendications précédentes, **caractérisée en ce que** les fibres ont un facteur de forme (L/D) allant de 5 à 150.

10. Utilisation selon l'une quelconque des revendications précédentes, **caractérisée en ce que** les fibres ont un titre allant de 0,15 à 30 deniers.

11. Utilisation selon l'une quelconque des revendications précédentes, **caractérisée en ce que** les fibres sont choisies parmi les fibres de soie, de coton, de laine, de lin, de cellulose extraites notamment du bois, des légumes ou des algues, de polyamide (Nylon® ), de cellulose modifiée, de poly-p-phénylène téréphtalamide, en acrylique, de polyoléfine, de verre, de silice, d'aramide, de carbone, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

**12.** Utilisation selon l'une quelconque des revendications précédentes, **caractérisée en ce que** les fibres sont enrobées.

**13.** Utilisation selon l'une quelconque des revendications précédentes, **caractérisée en ce que** les fibres sont choisies parmi les fibres de polyamide, les fibres de poly-p-phénylène téréphtalamide, les fibres de coton et leurs mélanges.

**14.** Utilisation selon l'une quelconque des revendications précédentes, **caractérisée en ce que** les fibres sont présentes en une quantité allant de 0,1 à 30 % en poids par rapport au poids total de la composition.

**15.** Utilisation selon l'une quelconque des revendications précédentes, **caractérisée en ce que** la composition comprend un milieu physiologiquement acceptable.

**16.** Utilisation selon l'une quelconque des revendications 3 à 15, **caractérisée en ce que** la composition constitue un produit de maquillage.

**17.** Utilisation selon l'une quelconque des revendications 3 à 15, **caractérisée en ce que** la composition est un produit coulé.

**18.** Procédé de traitement cosmétique en vue de matifier, lisser et/ou uniformiser le teint, et/ou d'estomper les microreliefs, les rides, les ridules, les pores de la peau, comprenant l'application sur la peau, d'une composition cosmétique de soin de la peau, contenant des fibres dans un milieu physiologiquement acceptable.

**19.** Procédé de maquillage des microreliefs, rides, ridules et/ou pores de la peau, consistant à appliquer sur la peau, une composition cosmétique contenant des fibres dans un milieu physiologiquement acceptable.

**20.** Procédé selon la revendication 18 ou 19, **caractérisé en ce que** la composition est une émulsion.

**21.** Composition cosmétique contenant des fibres et au moins un actif de traitement des peaux grasses.

**22.** Composition selon la revendication précédente, **caractérisée en ce que** l'actif est choisi parmi les dérivés de b-lactame, les dérivés de quinolone, la ciprofloxacine, la norfloxacine, la tétracycline et ses sels, l'érythromycine et ses sels, l'amikacine et ses sels, le 2,4,4'-trichloro-2'-hydroxy diphényl éther (ou triclosan), le 3,4,4'-trichlorocarbanilide (ou triclocarban), le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamidine et ses sels, la gentamicine et ses sels, la kanamycine et ses sels, la linéomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amanfadine, le para-chloro-méta-xylénol, la nystatine, le tolnaftate, l'acide 3-hydroxy benzoïque et ses sels, l'acide 4-hydroxy benzoïque et ses sels, l'acide 2-hydroxybutanoïque et ses sels, l'acide 2-hydroxypentanoïque et ses sels, l'acide 2-hydroxyhexanoïque et ses sels, l'acide phytique et ses sels, l'acide N-acétyl-L-cystéine et ses sels, l'acide lipoïque et ses sels, l'acide azélaïque et ses sels, l'acide arachidonique et ses sels, l'ibuprofène, le naproxène, l'hydrocortisone, l'acétominophène, le résorcinol, l'octopirox, le chlorhydrate de lidocaïne, le clotrimazole, l'acide 10-hydroxy-2-décanoïque et ses sels, les sels de zinc, les extraits végétaux de la famille des éricacées, les extraits végétaux riches en soufre, et leurs mélanges.

**23.** Composition selon la revendication 21 ou 22, **caractérisée en ce que** la quantité d'actif(s) va de 0,0001 à 30 % en poids et de préférence de 0,001 à 20 % en poids par rapport au poids total de la composition.

**24.** Composition selon l'une quelconque des revendications 21 à 23, **caractérisée en ce que** les fibres ont une longueur (L) allant de 1 μm à 10 mm.

**25.** Composition selon l'une quelconque des revendications 21 à 24, **caractérisée en ce que** les fibres ont une section comprise dans un cercle de diamètre (D) allant de 1 nm à 100 μm.

26. Composition selon l'une quelconque des revendications 21 à 25, **caractérisée en ce que** les fibres ont un facteur de forme (L/D) allant de 5 à 150.

27. Composition selon l'une quelconque des revendications 21 à 26, **caractérisée en ce que** les fibres sont choisies parmi les fibres de soie, de coton, de laine, de lin, de cellulose extraites notamment du bois, des légumes ou des algues, de polyamide (Nylon®), de cellulose modifiée, de poly-p-phénylène téréphtalamide, en acrylique, de polyoléfine, de verre, de silice, d'aramide, de carbone, de Téflon® , de collagène insoluble, de polyesters, de polychlorure de vinyle ou de vinylidène, d'alcool polyvinylique; de polyacrylonitrile, de chitosane, de polyuréthane, de polyéthylène phtalate, des fibres formées d'un mélange de polymères, les fibres synthétiques résorbables, et leurs mélanges.

28. Composition selon l'une quelconque des revendications 21 à 27, **caractérisée en ce que** les fibres sont choisies parmi les fibres de polyamide, les fibres de poly-p-phénylène téréphtalamide, les fibres de coton et leurs mélanges.

29. Composition selon l'une quelconque des revendications 21 à 28, **caractérisée en ce que** les fibres sont présentes en une quantité allant de 0,1 à 30 % en poids par rapport au poids total de la composition.

30. Composition selon l'une quelconque des revendications 21 à 29, **caractérisée en ce qu'**elle est sous forme d'une émulsion.

31. Utilisation cosmétique de la composition selon l'une quelconque des revendications 21 à 30 pour réguler l'aspect huileux de la peau et/ou traiter les peaux grasses.